# EUROPEAN PATENT APPLICATION

(11) **EP 2 078 757 A1**
(43) Date of publication of application: **15.07.2009**
(21) Application number: 07122763.1
(22) Date of filing: 10.12.2007
(51) Int. Cl.: C12Q 1/68

(54) **New labels for surface-enhanced resonant Raman spectroscopy**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

The present invention is related to the use of an oligonucleotide labelled with a label suitable for SERRS, wherein the label has a negative charge and the oligonucleotide is not modified to compensate the negative charge.

## Description

### FIELD OF THE INVENTION

The present invention relates to labels for use in Surface-enhanced (resonant) Raman (SE(R)RS) and methods for their use.

### BACKGROUND OF THE INVENTION

Surface-enhanced (resonant) Raman (SE(R)RS) is a technique for sensitive and selective detection and identification of molecules adsorbed at a roughened metal surface. Raman scattering can be enhanced by several orders of magnitude. In SERRS it is possible to combine the sensitivity of molecular resonance (by a specific dye) with the sensitivity of surface-enhanced Raman scattering (SERS) so that very low concentrations of an analyte can be measured. The technique can e.g. be applied in molecular diagnostics to identify deoxyribonucleic acid (DNA) of pathogenic bacteria or proteins involved in infectious diseases. In this area a rapid, specific and highly sensitive identification is crucial for effective treatment. Optical methods, especially fluorescence spectroscopy, are widely used to identify certain biomolecules. SERRS has the unique feature that the scattered light consists of sharp, molecule-specific vibrational bands which makes discrimination of multiple analytes possible. Various articles are published on DNA identification by Surface-Enhanced (Resonant) Raman Spectroscopy [e.g. Isola et al. (1998) Anal. Chem. 70, 1352-1356; Deckert et al. (1998) Anal. Chem. 70, 2646-2650; Graham et al. (2000) Biopolymers 57, 85-91; Faulds et al. (2004) Anal. Chem. 76, 592-598].

The presence of a certain targeted DNA sequence can be measured via the SERRS signal of a SERRS active label attached to the DNA sequence. Simultaneous detection of multiple labels (and thereby multiple DNA sequences) in solution by SERRS has been demonstrated. Multiplexing of 6 has recently been performed (Faulds et al. "Highly multiplexed detection of labelled oligonucleotides using surface enhanced resonance Raman scattering (SERRS)" submitted).

To obtain resonance enhancement of the labels, the excitation wavelength needs to overlap with the electronic absorption band of the label. For surface enhancement the label needs to adsorb at a roughened metal surface. Aggregated colloidal particles have been found to be practical examples of roughened metal surfaces. In solution, these colloidal particles carry a certain charge. Typically, citrate or EDTA-reduced silver colloids are used, which are negatively charged.

Currently, the common understanding is that a positively charged label will easily adsorb at the silver surface to ensure surface enhancement. Negatively charged labels, however, will not adsorb at the surface due to electrostatic repulsion and are therefore considered inappropriate for use as such as SERRS labels. When a negatively charged label is used in SERRS, a modification is introduced in the attached nucleic acid probe to create an area of positive charge, which then enables adsorption of the label onto the metal surface. Propargylamine modification of DNA has been introduced to get the negatively charged dyes HEX, TET, FAM to the surface of the metallic colloidal particles (Faulds et al, (2005) Analyst 130, 1125-1131, US 6,127,120). The use of negatively charged dyes in SERRS accordingly requires tailor made synthesis of labelled DNA probes with additional 5-(1-propargylamino)-2'-deoxyuridine nucleotides.

Faulds et al. (2005) in Talanta 67, 667-671 describe the use of unmodified DNA labelled with Bodipy TR-X, a compound without an ionic charge which carries a thiophene function considered to promote surface binding.

For multiplexing, the choice of commercially available positively charged labels that not only adsorb without any modification at the negatively charged metal surface but also have similar absorption spectra, so as to meet the resonance condition upon single wavelength excitation, is limited.

Particular and preferred aspects of the invention are set out in the accompanying independent and dependent claims. Features from the dependent claims may be combined with features of the independent claims and with features of other dependent claims as appropriate and not merely as explicitly set out in the claims.

### SUMMARY FO THE INVENTION

The present invention relates to dyes suitable for SERRS which, despite their negative charge, have been found to be suitable as direct labels of oligonucleotides. It has been established that the oligonucleotides need not contain a positive charge to ensure adequate signal detection of a negatively charged label. This opens up the number of dyes/labels which can be used in SERRS multiplexing experiments.

Accordingly, one aspect of the present invention relates to the use of probes labelled with negatively charged dyes in SERRS detection, without modifying the charge of the oligonucleotide. More specifically, the invention relates to the use of oligonucleotides labelled with a label suitable for SERRS, characterised in that the oligonucleotide essentially consists of bases with neutral nucleosides, and the label has a negative charge. Preferably the oligonucleotide consists of bases with neutral nucleosides, and the label has a negative charge

In particular embodiments the negatively charged label is a fluorescent label, more particularly a fluorescent label with an absorption maximum between 490 and 544 nm.

In further particular embodiments oligonucleotides used in the context of the invention consist of nucleotides with unmodified nucleosides, more particularly the oligonucleotide does not comprise 5-(1-propargylamino)-2'-deoxyuridine.

In further particular embodiments, a negatively charged fluorescent label as used herein is a label selected from the group consisting of ATTO 532, HEX, FAM, TET, Yakima Yellow, Eosin, Erythrosin, Dicholorofluorescein, Tetrabromosulfonefluorescein, JOE, Alexa Fluor 488 and 514.

Another aspect of the invention relates to methods which involve the use of negatively charged dyes in accordance with the invention. More particularly, methods are provided for detecting one or more nucleic acid targets in a sample by surface-enhanced resonance Raman scattering (SERRS), preferably with high reproducibility and high signal intensity, making use of labeled oligonucleotides as described herein. In particular embodiments methods are provided which comprise the steps of:
(a) Contacting the sample with an oligonucleotide with a label suitable for SERRS detection, wherein:
(b) Contacting the probe with a metal surface that is preferably roughened, and
(c) Detecting the SERRS signal generated by the label on the oligonucleotide, wherein the methods are characterized in that the labelled oligonucleotide essentially consist of bases with neutral nucleosides, and the label suitable for SERRS detection has a negative charge.

In particular embodiments methods described herein, the label suitable for SERRS detection is a fluorescent label.

In further particular embodiments methods are provided wherein the labelled oligonucleotide is a target-specific oligonucleotide.

In yet further particular embodiments of methods described herein, oligonucleotides are used which consist of nucleotides with unmodified nucleosides, more particularly, the oligonucleotide does not comprise 5-(1-propargylamino)-2'-deoxyuridine.

In particular embodiments methods are provided which are methods for detecting multiple targets in a sample, wherein at least two different oligonucleotides each labelled with a different label are contacted with the sample and the detection step comprises detecting the SERRS signal generated by each of the different labels, which methods are characterized in that at least one oligonucleotide with a negatively charged label is used. More particularly, at least one of the oligonucleotides is characterised in that the oligonucleotide consists of bases with neutral nucleosides, and the label has a negative charge.

In further particular embodiment of methods described herein using more than one label, detection of the SERRS signal of the different labels is performed at one wavelength.

In particular embodiments of multiplexing methods described hereinabove, the labels used have an absorption maximum between 490 and 544 nm.

In particular embodiments methods of detection described hereinabove making use of at least one oligonucleotide with a negatively charged label as described herein, which methods are performed with at least 5 different fluorescent labels, more particularly with labels selected from the group consisting of Atto 520, Atto 532, Atto 540Q, Alexa 488, Alexa 514, Alexa 532, BODIPY 530/550, BODIPY TMR-X, BODIPY R6G, BODIPY FL, FAM, HEX, JOE, Rhodamine 100/110, Rhodamine 6G, Rhodamine Green, TET, TAMRA, Erythrosin, Eosin, Dichlorofluorescein, and Cy3. In further particular embodiments at least 6, more particularly at least 7, most particularly 8, 9 or 10 different labels, optionally selected from the list described above are used.

The above and other characteristics, features and advantages of the present invention will become apparent from the following detailed description, taken in conjunction with the accompanying Figures, which illustrate, by way of example, the principles of the invention. This description is given for the sake of example only, without limiting the scope of the invention. The reference Figures quoted below refer to the attached drawings.

### BRIEF DESCRIPTION OF THE DRAWINGS

Fig. 1 shows the SERRS spectrum of unmodified DNA labelled with the negatively charged fluorescent dye FAM. Excitation is performed at 532 nm. Concentration of the label in the sample is 2.5 x 10⁻¹⁰ M.
Fig. 2 shows absorption spectra of 8 labels known as fluorescent dyes, linked to unmodified DNA. The peak around 260 nm is due to absorption by DNA and around 500/550 nm to absorption by the dye. The resonance conditions for SERRS are met (see overlap of excitation wavelength) at 514 or 532 nm.
Fig. 3 shows the SERRS spectra of 8 labels know as fluorescent dyes linked to unmodified DNA, upon excitation with 514 nm. Concentration of the label in the sample is 1 x 10⁻¹⁰ M.

In the different Figures, the same reference signs refer to the same or analogous elements.

### DETAILED DESCRIPTION OF THE INVENTION

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

Essentially consist of bases with neutral nucleosides means that most, prferably all bases are neural nucleosides. The term **"label"** or **"dye",** as used herein, refers to a molecule or material capable of generating a detectable signal. Labels which are **SERRS active** or **'suitable for SERRS'** are labels which are capable of generating a SERRS spectrum when appropriately illuminated. In order to be SERRS active a dye must be polarisable upon excitation, and be able to come into close contact (distance <30 nm) with a rough metal surface (e.g. aggregated (noble) metal colloidal particles).

The term **"negatively charged"** as used herein when referring to a dye, relates to the fact that the charge of the dye in the buffer wherein SERRS measurements takes place is negative as determined by methods described herein.

The present invention is based on the finding that, contrary to what is reported in the literature, labels suitable for SERRS which are negatively charged, can be used for SERRS detection without the need to compensate the charge, e.g. by adding positively charged nucleosides to the probe used for detection.

Accordingly, one aspect of the invention relates to the use of oligonucleotides labelled with a label suitable for SERRS, wherein the label has a negative charge and wherein the oligonucleotide essentially consists of bases with neutral nucleosides. Thus this aspect relates to the use of SERRS labels with a negative charge directly linked to an unmodified probe. Particular embodiments relate to the use of labels, which are known as fluorescent labels in SERRS, irrespective of their charge. In particular embodiments, the invention describes the use of fluorescent labels which are known to be suitable for SERRS, as labels for oligonucleotides without further modification of the probe to compensate for the negative charge.

SERRS active labels are known in the art. The criteria for determining whether or not a dye is suitable for SERRS are known by the skilled person. WO-A-2006/066180 describes structural criteria for identifying a label suitable for SE(R)RS. Generally, a dye is suitable for SERRS when it fulfils the following functional criteria:

The dye is Raman active, which requires that the polarisability of the dye changes upon excitation, thus creating a change in charge distribution during the molecular vibration. This can be experimentally verified by measuring normal Raman spectra of the dye under non-resonant excitation conditions (when using resonance excitation conditions fluorescence of the label hampers normal Raman measurements; in SERRS measurements the fluorescence is quenched by the metal surface).

The dye is capable of getting into close contact (distance <30 nm) with a metal surface, e.g. aggregated (noble) metal colloidal particles; if the distance of the molecule to the surface is too large the fluorescence is not quenched.

The orientation of the dye with respect to the metal surface should be such that the electronic transition moment (dipole moment) is not parallel to the metal surface. Polarisation sensitive SERRS can give information on the orientation of the molecules at the metal surface.

Whether or not a dye is SERRS active can be easily tested by measuring the SERRS spectrum.

The charge of a dye (i.e. whether it is negative, neutral or positive) is influenced by the pH of the buffer in which the dye is present. In the context of the present invention, the relevant conditions are those of the buffer wherein the SERRS measurements take place (typically between pH 6.5 and 9.0). The charge of dye itself, i.e. not coupled to an oligonucleotide, can be determined e.g. by assaying the movement of the dye in a gel or matrix in an electric field.

Upon labelling of an oligonucleotide with a dye, a functional group on the dye is used to react the dye with the oligonucleotide. This may affect the charge of the dye. Where a charged group of a dye is used to react with an oligonucleotide, the loss of a functional group and the effect of this loss on the charge of the dye is assessed either on a theoretical basis or by measuring the charge of a modified version of the dye wherein the functional group of the dye which is used for the oligonucleotide labelling, is modified. Contrary to the generally applied practice, the present invention discloses that SERRS measurements of oligonucleotide probes labelled with a negatively charged dye, more particularly a negatively charged fluorescent dye, can be performed without incorporation of positive charges into the oligonucleotide probe. Such incorporation of positive charges was typically achieved by incorporating propargylamine-modified nucleotides to an oligonucleotide probe (typically a tail of 12 nucleotides with alternating cytosine and propargylamine-modified deoxyuridine). Surprisingly, the present invention discloses that such modification is however not necessary in order to use a oligonucleotide probe with a negatively charged dye in SERRS measurement.

Particular embodiments of the invention relate to labelled oligonucleotides for use in SERRS which do not comprise nucleotides with positively charged nucleosides (such as 5-(1-propargylamino)-2'-deoxyuridine, abbreviated in the prior art as "7"), but comprise or consist of nucleotides with uncharged nucleosides. More particularly, the first 3, 6, 9 or up to 12 nucleotides adjacent to the dye are nucleotides with unmodified nucleosides. In particular embodiments, the oligonucleotide consists of unmodified A, C, T, and G nucleotides (DNA) or unmodified A, C, U and G nucleotides (RNA). The modification as referred to herein do not refer to the modification of the 5' or 3' end of the oligonucleotide which is used for coupling an oligonucleotide with a dye, but rather to the presence of additional charged nucleotides.

Particular embodiments of the present invention relate to the use of negatively charged fluorescent SERRS active dyes. A large number of fluorescent dyes have been found to be SERRS active.

A non-limiting list of examples of negatively charged fluorescent SERRS active dyes, and their absorption maximum, which are suitable as SERRS dyes are ATTO 532 (530 nm), Alexa Fluor dyes such as Alexa 488 (494 nm), 514 (517 nm), 546 (554 nm), HEX (537 nm), JOE (520 nm), Erythrosin (530 nm), Eosin (524 nm), Dichlorofluorescein (510 nm), Resorfurin (578 nm), Carbazine (720 nm), Tetrabromosulfonefluorescein (528 nm), FAM (494 nm), TET (519 nm), Yakima Yellow (526 nm) (Table 1).

**Table 1. Exemplary negatively charged fluorescent dyes**

| **Dye/label** | **Absorption maximum** | **Charge** |
|---|---|---|
| FAM | 494 nm | Negative |
| Alexa Fluor® 488 | 494 nm | Negative |
| Dichlorofluorescein | 510 nm | Negative |
| Alexa Fluor® 514 | 517 nm | Negative |
| TET | 519 nm | Negative |
| JOE | 520 nm | Negative |
| Eosin | 524 nm | Negative |
| Yakima Yellow | 526 nm | Negative |
| Tetrabromosulfonefluorescein | 528 nm | Negative |
| Erythrosin | 530 nm | Negative |
| Atto 532 | 532 nm | Negative |
| HEX | 537 nm | Negative |
| Alexa Fluor® 546 | 554 nm | Negative |
| Resorfurin | 578 nm | Negative |
| Carbazine | 720 nm | Negative |

In particular embodiments, the invention relates to the use of negatively charged SERRS active labels having an absorption maximum between 490 and 544 nm of which the electronic absorption band overlaps with and excitation wavelength of 514 or 532 nm. A non-limiting list of examples hereof includes TET, Yakima Yellow, FAM, JOE, HEX, Atto 532, Alexa Fluor 488 and 514.

As indicated above, particular embodiments of the invention relate to the use of SERRS active fluorescent dyes. Fluorescent dyes are available from different companies (Invitrogen, Molecular Probes, Atto-Tech etc.).

In further embodiments, the present invention provides for the use of dyes which are SERRS active dyes which are not fluorescent. It is the contribution of the present invention to allow the development of further SERRS active dyes without having to take the resulting charge of the dye into account.

Methods for coupling labels or dyes to oligonucleotides are known in the art. Oligonucleotides coupled to SERRS active fluorescent dyes are commercially available from e.g. Oswel (UK), Eurogentec (Belgium), IBA (Germany) and other companies.

Another aspect of the present invention relates to methods for detecting a nucleic acid target in a sample by surface-enhanced resonance Raman scattering (SERRS) with high reproducibility and high signal intensity. In these methods a sample is contacted with a target-specific oligonucleotide probe with a label suitable for SERRS detection, which is negatively charged as described above. The methods of the present invention involve oligonucleotide probes which essentially carry nucleosides which in sum have a neutral charge, and which carry a SERRS active label with a negative charge. In particular embodiments, the oligonucleotide probes consist of bases with neutral nucleosides. In further particular embodiments, the oligonucleotide probes used in these methods consist of nucleotides with unmodified nucleosides. Most particularly the probes do not comprise 5-(1-propargylamino)-2'-deoxyuridine. Particular embodiments of the methods of the invention make use of fluorescent SERRS active dyes which are negatively charged.

In particular embodiments methods according to the invention comprise the steps of contacting a sample with an oligonucleotide probe labelled as described herein, contacting the (bound or unbound) labelled oligonucleotide probe with a preferably roughened metal surface, and detecting the SERRS signal generated by the label on the probe.

An important application of the methods of the present invention is multiplexing, as the invention provides a wider variety of labels suitable for detection at the same wavelength.

Accordingly in particular embodiments, methods are provided for detecting multiple targets in a sample, wherein at least two different oligonucleotide probes each labelled with a different label are contacted with a sample and the detection step comprises detecting the SERRS signal generated by each of the different fluorescent labels. In these multiplexing methods, at least one of the oligonucleotide probes is labelled with a negatively charged SERRS active label and consists of bases with neutral nucleosides. In further particular embodiments two or more of the probes comprise a SERRS label with a negative charge (whereby the oligonucleotide probe itself consists of bases with neutral nucleosides). In further particular embodiments a combination of probes with negatively charged SERRS active dyes and positively or neutrally charged SERRS active dyes are used so as to obtain an optimal number of different dyes (and thus targets) which can be detected simultaneously.

In further particular embodiments, methods are provided which make use of SERRS active fluorescent dyes. Non-limiting examples of SERRS active labels include fluorescein dyes, such as 5- (and 6-) carboxy-4',5'-dichloro-2',7'-dimethoxy fluorescein, 5-carboxy-2',4',5',7'-tetrachlorofluorescein and 5-carboxyfluorescein; rhodamine dyes such as 5- (and 6-) carboxy rhodamine, 6-carboxytetramethyl rhodamine and 6-carboxyrhodamine X, phthalocyanines such as methyl, nitrosyl, sulphonyl and amino phthalocyanines, azo dyes such as those listed in US Pat. no. 6127120, azomethines, cyanines and xanthines such as the methyl, nitro, sulphano and amino derivatives, and succinylfluoresceins. Each of these may be substituted in any conventional manner, giving rise to a large number of useful labels. An overview of exemplary fluorescent labels envisaged in the context of the present invention is provided in Table 2. As indicated above, the present invention provides methods wherein at least one negatively charged fluorescent dye is used.

More particular embodiments of the invention relate to multiplexing using two or more, more particularly all eight of the following labels: Atto 520, Atto 532, Alexa 532, Bodipy 530/550, Bodipy TMRX, Bodipy R6G, Bodipy FL and Rhodamine Green. In further particular embodiments multiplexing is performed using 10 labels with a single excitation wavelength. In particular embodiments, methods of the invention involve the use of Rhodamine 6G and TAMRA in addition to the 8 above-mentioned dyes. Other combinations of commercially available dyes are envisaged which enable a wide range of multiplexing without special design of the labels. The methods of the present invention allow a very low limit of detection (20 pM).

**Table 2: list of Exemnlarv SERRS active fluorescent dyes**

| | **Absorption maximum** | **Charge** |
|---|---|---|
| FAM | 494 nm | Negative |
| Alexa Fluor® 488 | 494 nm | Negative |
| BODIPY® FL | 502 nm | Neutral |
| Rhodamine Green^{™}-X, mixed isomers | 503 nm | Neutral |
| Rhodamine Green^{™}, mixed isomers | 504 nm | Neutral |
| Rhodamine 110/110X | 505 nm | Neutral |
| Dichlorofluorescein | 510 nm | Negative |
| Alexa Fluor® 514 | 517 nm | Negative |
| TET | 519 nm | Negative |
| Atto 520 | 520 nm | Positive |
| JOE, 6-isomer | 520 nm | Negative |
| Eosin | 524 nm | Negative |
| Carboxy-rhodamine 6G, 6-isomer (R6G-6) | 524 nm | Positive |
| Carboxy-rhodamine 6G, 5-isomer (R6G-5) | 524 nm | Positive |
| Alexa Fluor® 532 | 525 nm | Neutral |
| Yakima Yellow | 526 nm | Negative |
| BODIPY® R6G | 528 nm | Neutral |
| Tetrabromosulfonefluorescein | 528 nm | Negative |
| Erythosin | 530 nm | Negative |
| Atto 532 | 532 nm | Negative |
| BODIPY® 530/550 | 534 nm | Neutral |
| HEX | 537 nm | Negative |
| Atto 540Q | 542 nm | Structure not available |
| BODIPY®TMR-X | 544 nm | Neutral |
| Cy3 | 552 nm | Neutral |
| Alexa Fluor ® 546 | 554 nm | Negative |
| TAMRA | 565 nm | Positive |
| Resorfurin | 578 nm | Negative |
| ROX | 585 nm | Neutral |

One of the well-known advantages of SERRS over the commonly used fluorescence detection is exactly that it allows the simultaneous differential detection of a number of different labels, due to the much sharper (vibrational) spectral features of SERRS as compared to (electronic) broad features in fluorescence. A further advantage of multiplexing using SERRS is that with well-chosen labels, only a single excitation wavelength can be used, whereas in fluorescence for each label a different excitation wavelength needs to be used.

Labels experimentally found to be suitable for SERRS include Atto 520, Atto 532, Atto 540Q, Alexa 488, Alexa 514, Alexa 532, BODIPY 530/550, BODIPY TMR-X, BODIPY R6G, BODIPY FL, FAM, HEX, JOE, Rhodamine 100/110, Rhodamine 6G, Rhodamine Green, TET, TAMRA, Erythrosin, Eosin, Dichlorofluorescein, and Cy3.

In a particular embodiments multiplexing can be performed with fluorescent labels with an absorption maximum between 490 and 544. For this purpose fluorescent labels can be used selected from the group consisting of Atto 520, Atto 532, Alexa 532, FAM, HEX, TET, JOE, BODIPY 530/550, BODIPY TMR-X, BODIPY R6G, BODIPY FL, Rhodamine Green, or any other fluorescent label with an absorption maximum between 490 and 544 indicated in Table 2. For these labels, excitation wavelengths of 514 or 532 nm can be used.

Using the methods and tools of the present invention which allow the use of negatively labelled dyes without the need to modify the probes, multiplexing of an assay can be significantly increased.

Suitable methods for detecting SERRS signals are known to the skilled person. The detection of dyes attached to DNA is described *in extenso* in e.g. the above-cited publications of Faulds et al.. The present invention provides for a general principle of detecting DNA using probes labelled with a SERRS active dye. The specific methods of detection, i.e. the nature of the assay is not critical.

Typically, methods for detecting a target involve the use of a target-specific probe. In particular embodiments a labelled oligonucleotide probe is used which hybridises specifically with a sequence of the target. Alternatively indirect detection methods can be used wherein the labelled oligonucleotide is a molecule which competes with the target for the binding to a probe. In further particular embodiments the oligonucleotide is a probe which hybridises specifically with a sequence within an oligonucleotide, which oligonucleotide further also comprises a sequence capable of hybridising specifically with the target. These are non-limiting examples of detection principles which can be used in the context of the present invention.

The use of oligonucleotides in methods described hereinabove is characterised in that there is no need to modify these oligonucleotides by introduction of a positive charge to ensure interaction of the label which is a SERRS active dye with a SERRS active surface. This implies that probes can be designed such that only the ability to ensure detection of the target and the ability to attach a label need to be taken into account. In particular embodiments, the labelled oligonucleotide consists of bases with neutral nucleosides. As detailed above, if a (charged) functional group of a nucleoside is used for attaching the label, this need not be taken into account in the context of the present invention (as it is no longer present in the labelled oligonucleotide).

Labelled oligonucleotides used in the context of the present invention are oligonucleotides carrying one or more labels. These labels may be identical (e.g. for signal enhancement) or different (e.g. to allow double detection of the signal). In particular embodiments, the oligonucleotides carry one label.

The concentration of the oligonucleotide (and the dye where single-labelled oligonucleotides are used) in methods of the invention can vary, and will typically (but not necessarily) depend on the concentration of analyte to be detected. Since SERRS is particularly suited for measuring low concentrations of analyte (e.g. lower than 1 nM), according to a particular embodiment of the invention, the labelled oligonucleotide/SERRS active dye is used at a concentration lower than 1x10⁻⁹ M.

Specific reagents suitable for use in methods involving SERRS detection are described in the art. More particularly, different conditions preparing metallic colloidal solutions and different buffer systems for performing SERRS measurements are described in the art.

Buffers used for SERRS generally have a pH between 7 and 9 and include a polyamine as aggregating agent. Exemplary buffers used in SERRS are Tris/HCl buffers between pH 7 and 9.

Methods of the present invention are suitable for the detection of different types of nucleic acids, for instance DNA such as a gene, viral DNA, bacterial DNA, fungal DNA, mammalian DNA, or DNA fragments. Methods of the present invention are similarly suitable for the detection of analytes which are RNA such as viral RNA, mRNA, rRNA. The analyte can also be cDNA, oligonucleotides, or synthetic DNA, RNA, PNA, synthetic oligonucleotides, modified oligonucleotides or other nucleic acid analogues. An analyte may comprise single-stranded and double-stranded nucleic acids. An analyte may, prior to detection, be subjected to manipulations such as digestion with restriction enzymes, copying by means of nucleic acid polymerases (e.g. PCR), shearing or sonication thus allowing fragmentation to occur.

The term "sample" is used in the context of the present invention in a broad sense and is intended to include a wide range of biological materials as well as compositions derived or extracted from such biological materials. The sample may be any suitable preparation in which the analyte is to be detected. The sample may comprise, for instance, a body tissue or fluid such as but not limited to blood (including plasma and platelet fractions), spinal fluid, mucus, sputum, saliva, semen, stool or urine or any fraction thereof. Exemplary samples include whole blood, red blood cells, white blood cells, buffy coat, hair, nails and cuticle material, swabs, including but not limited to buccal swabs, throat swabs, vaginal swabs, urethral swabs, cervical swabs, rectal swabs, lesion swabs, abcess swabs, nasopharyngeal swabs, and the like, lymphatic fluid, amniotic fluid, cerebrospinal fluid, peritoneal effusions, pleural effusions, fluid from cysts, synovial fluid, vitreous humor, aqueous humor, bursa fluid, eye washes, eye aspirates, plasma, serum, pulmonary lavage, lung aspirates, biopsy material of any tissue in the body. The skilled artisan will appreciate that lysates, extracts, or material obtained from any of the above exemplary biological samples are also considered as samples. Tissue culture cells, including explanted material, primary cells, secondary cell lines, and the like, as well as lysates, extracts, supernatants or materials obtained from any cells, tissues or organs, are also within the meaning of the term biological sample as used herein. Samples comprising microorganisms and viruses are also envisaged in the context of analyte detection using the methods of the invention. Materials obtained from forensic settings are also within the intended meaning of the term sample. Samples may also comprise foodstuffs and beverages, water suspected of contamination, etc. These lists are not intended to be exhaustive.

Detection by surface-enhanced spectroscopies such as surface-enhanced resonance Raman spectroscopy (SERRS) is based on the strong enhancement of Raman scattering observed for analytes adsorbed onto an appropriate 'SERRS active surface'. Typically, the surface is a noble (Au, Ag, Cu) or alkali (Li, Na, K) metal surface. Generally, the SERRS active surface is provided as an aggregation of metal colloid particles, which typically results in enhancements for SERRS of greater than 10⁸-10¹² of the Raman scattering. The colloidal suspension of metal particles is for example a colloidal suspension of silver particles, such as citrate reduced silver particles.

Where metal nanoparticles are used as a SERRS active surface; these may be a naked metal or may comprise a metal oxide layer on a metal surface. They may include an organic coating such as of citrate or of a suitable polymer, such as polylysine or polyphenol, to increase its sorptive capacity. Metal colloid particles making up a SERRS active surface are typically colloidal nanoparticles aggregated in a controlled manner so as to be of a uniform and desired size and shape and as stable as possible against self-aggregation. Processes for preparing such colloids are well known (e.g. described in US Application No. 20050130163). Alternative methods of preparing nanoparticles have also been described (e.g. U.S. Pat. Nos. 6054495, 6127120, 6149868). Nanoparticles may also be obtained from commercial sources (e.g. Nanoprobes Inc., Yaphank, N.Y.; Polysciences, Inc., Warrington, Pa.). Metal particles for use in this context can be of any size as long as they give rise to a SERRS effect. Typically they have a diameter of about 4-50 nm, most particularly between 25 nm and 40 nm, depending on the type of metal. Methods for obtaining metal particles having a diameter of 40 nm have been described such as by Munro et al. (1995) Langmuir 11, 3712-3720.

Addition of colloid metal particles to a suspension of labelled oligonucleotide results in the adsorption of the label to the metal surface. Hereafter, aggregation of the colloids is typically achieved by addition of a polyamine. Suitable polyamines include monomeric or polymeric polyamines, such as spermine or spermidine, 1,4-diaminopiperazine, diethylenetriamine, N-(2-aminoethyl)-1,3-propanediamine, triethylenetetramine and tetraethylenepentamine. Alternatively, salts like NaCl can be used for aggregation.

Particular embodiments of methods of the invention involve the application of SERRS, and operating at the resonant frequency of a SERRS active label gives increased sensitivity. In this case, the light source used to generate the Raman spectrum is a coherent light source, e.g. a laser, tuned substantially to the maximum absorption frequency of the label being used. This frequency may shift slightly on association of the label with the SERRS active surface (and the analyte and/or analyte binding species), but it is within the skill of the artisan to tune the light source to accommodate this. The light source may be tuned to a frequency in the absorption band, or near to the label's absorption maximum, or to a frequency at or near that of a secondary peak in the label's absorption spectrum. Simultaneously, by tuning the frequency of the light source in the absorption band of the aggregated colloids, plasmons are excited in the colloidal aggregates that contribute to the enhancement of the electrical fields between the aggregated particles. Excitation of the dye molecules at the interstitial spaces between the colloid particles, the so-called hot spots, gives largest enhancement of the SERRS signals.

In SERRS detection typically the fingerprint spectrum is measured in order to identify each label. However, if the different labels used each have a unique spectral line, then it can be sufficient to detect signal intensity at a chosen spectral line frequency or frequencies.

Typically, the detection step in the SERRS based detection methods of the present invention are carried out using incident light from a laser, having a frequency in the visible spectrum. The exact frequency chosen will depend on the label, surface and analyte. Frequencies in the red area of the visible spectrum tend, on the whole, to give rise to better surface enhancement effects. However, it is possible to envisage situations in which other frequencies, for instance in the ultraviolet or the near-infrared ranges, might be used. The selection and, if necessary, tuning of an appropriate light source, with an appropriate frequency and power, will be well within the capabilities of one of ordinary skill in the art, particularly on referring to the available SERRS literature.

Excitation sources for use in SERRS-based detection methods include, but are not limited to, nitrogen lasers, helium-cadmium lasers, argon ion lasers, krypton ion lasers, etc. Multiple lasers can provide a wide choice of excitation lines which is critical for resonance Raman spectroscopy. According to a specific embodiment, an argon ion laser is used in a LabRam integrated instrument (Jobin Yvon) at an excitation of 514.5 nm.

Laser power, or excitation power, used in the context of the present invention can be varied, e.g. depending on the properties of the label used. According to a specific embodiment of the invention, the excitation power is between 1 and 15 mW.

An excitation beam suitable for use in the context of the present invention may be spectrally purified with a bandpass filter and may be focused on a substrate using an objective lens. The objective lens may be used to both excite the sample and to collect the Raman signal, by using a holographic beam splitter to produce a right-angle geometry for the excitation beam and the emitted Raman signal. The intensity of the Raman signal needs to be measured against an intense background from the excitation beam. The background is primarily Rayleigh scattered light and specular reflection, which can be selectively removed with high efficiency optical filters. For example, a holographic notch filter may be used to reduce Rayleigh scattered radiation.

Several objective lenses can be used in the context of the invention. According to a specific embodiment, the objective lens is a 5x, 10x, 20x, 50x or a 100x objective lens. In most set ups, the nature of the objective lens influences the excitation volume, since the focal distance, numerical aperture and working distance determine the volume irradiated. Accordingly, where objectives of 5x, 10x, and 50x are used, the excitation volume typically decreases from 5x to 50x, accordingly.

The surface-enhanced Raman emission signal may be detected by a Raman detector. A variety of detection units of potential use in Raman spectroscopy are known in the art and any known Raman detection unit may be used. An example of a Raman detection unit is disclosed e.g. in U.S. Pat. No. US 6002471. Other types of detectors may be used, such as a charge coupled device (CCD), with a red-enhanced intensified charge-coupled device (RE-ICCD), a silicon photodiode, or photomultiplier tubes arranged either singly or in series for cascade amplification of the signal. Photon counting electronics can be used for sensitive detection. The choice of detector will largely depend on the sensitivity of detection required to carry out a particular assay. Several devices are suitable for collecting SERRS signals, including wavelength selective mirrors, holographic optical elements for scattered light detection and fibre-optic waveguides.

Devices suitable for obtaining and/or analysing a SERRS spectrum may include some form of data processor such as a computer. Once a SERRS signal has been captured by an appropriate detector, its frequency and intensity data will typically be passed to a computer for analysis. Either the fingerprint Raman spectrum will be compared to reference spectra for identification of the detected Raman active compound or the signal intensity at the measured frequencies will be used to calculate the amount of Raman active compound detected.

Methods of the present invention include measuring the SERRS signal. The time used for detection of the SERRS spectrum has been found not to be a critical factor in ensuring low variability. Accordingly, the present invention envisages embodiments wherein the SERRS signal is measured for a time period between 0.1 and 300 seconds. According to particular embodiments, SERRS signals are measured for a time period of 100 seconds. According to other particular embodiments, SERRS signals are measured for a time period of between 0.1 and 10 seconds.

Other arrangements of the systems and methods embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

### Example 1 : Use of negatively charged FAM label in SERRS.

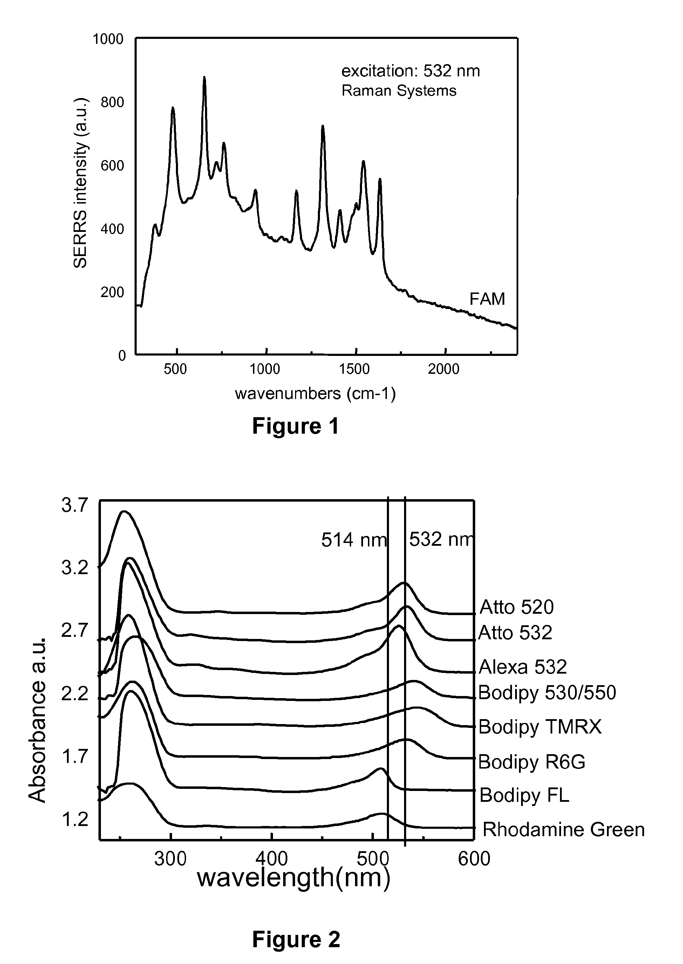

An oligonucleotide consisting of 25 unmodified nucleotides (i.e. with neutral nucleosides), was directly labelled with the negatively charged FAM dye (absorption maximum at 494 nm). The FAM labelled oligonucleotide was assayed as SERRS label. 250 pM SERRS probe was mixed with citrate reduced Ag colloids (prepared from a modified Lee-Meisel procedure, Munro et al. (1995) Langmuir 11, 3712-3720) and aggregated using spermine. Using a Raman Systems 3000HR spectrometer with an excitation wavelength of 532 nm, a strong SERRS signal was obtained (Figure 1).

This demonstrates that a negatively charged label can be used directly for SERRS detection, without modification of the probe to compensate for the charge of the label.

### Example 2. : Multiplexing using SERRS labels irrespective of their charge.

Eight commercially available dyes not previously used as SERRS labels were tested for use in SERRS multiplexing, irrespective of their charge: Atto 520 (positive), Atto 532 (negative), Alexa 532 (neutral), Bodipy 530/550 (neutral), Bodipy TMR-X (neutral), Bodipy R6G (neutral) and Rhodamine green (neutral).

Each of the labels was linked to an oligonucleotide, with no modifications apart from the reaction with the dye (IBA GmbH, Göttingen, Germany) with a length of 25-30 basepairs. SERRS measurements were performed using citrate-reduced silver colloids. Spermine was used as an aggregating agent.

For 532 nm excitation a device such as described in Example 1 was used. For 514 nm an excitation LabRam integrated instrument (Jobin Yvon) with an argon ion laser was used.

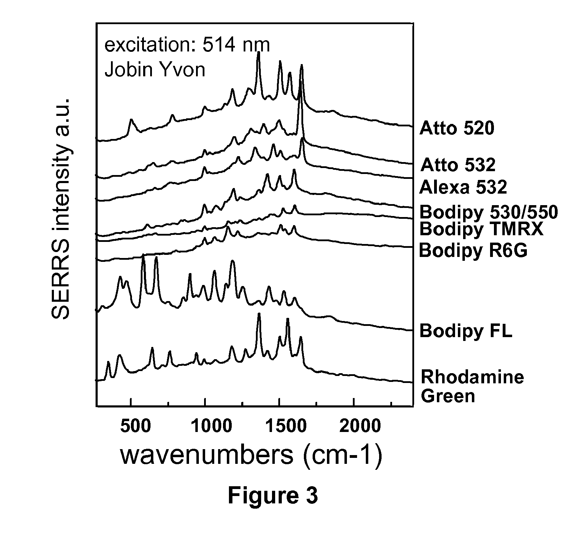

The results are shown in Figure 2. Though some of these dyes are more suitable for 514 nm excitation and others for 532 nm, all of the eight dyes tested give very distinct SERRS spectra upon excitation at 514 nm and 532 nm. The SERRS spectra of these labels upon excitation with 514 nm are illustrated in Figure 3. The concentration of the label in the sample is 1x10⁻¹⁰M.

Thus, all of the dyes fulfil resonance conditions to carry out multiplexing with a single excitation wavelength (green laser light, e.g. 514 or 532 nm), irrespective of their charge.

The detection limit of these dyes was further investigated upon excitation with 532 nm and 514 nm, respectively. The results are provided in Table 3

**Table 3: Detection limit (pM) of unmodified oligonucleotides labelled with fluorescent dyes in SERRS upon excitation with 532 nm and 514 nm, respectively**

| **Label** | **Excitation: 532 nm ##** | **Excitation: 514 nm ##** |
|---|---|---|
| Atto 520 | 6 | 32 |
| Atto 532 | 9 | 27 |
| Alexa 532 | 12 | 34 |
| Bodipy 530/550 | 6 | 35 |
| Bodipy TMRX | 9 | 46 |
| Bodipy R6G | 18 | 38 |
| Bodipy FL | 28 | 23 |
| Rhodamine GR | 52 | 27 |

| | | |
|---|---|---|
| ## Limit of Detection = 3 x RMSECV [pM] determined from partial least squares regression analysis, with 2 latent variables. | | |

As indicated in the table the limit of detection was defined as LoD = 3 x RMSECV [pM] determined from partial least squares regression analysis, with 2 latent variables In the prior art LoD is determined from a signal at a concentration which is 3 times larger than the background noise. Using this type of calculation, the obtained LoD values are in the femtomolar range. For the first 6 dyes the LoD is lower with 532 nm, since full resonance condition is met. For the last 2 dyes better resonance is obtained with 514 nm excitation.

## Claims

1. Use of an oligonucleotide labelled with a label suitable for SERRS in SERRS detection, **characterised in that:**
- the oligonucleotide essentially consists of bases with neutral nucleosides, and
- the label has a negative charge.

2. The use according to claim 1, wherein the label is a fluorescent label.

3. The use according to claim 2, wherein the fluorescent label has an absorption maximum between 490 and 544 nm.

4. Use according to claim 1, wherein the oligonucleotide consists of nucleotides with unmodified nucleosides.

5. Use according to claim 1, wherein the oligonucleotide does not comprise 5-(1-propargylamino)-2'-deoxyuridine.

6. The use according to claim 2, wherein the fluorescent label is selected from the group consisting of ATTO 532, HEX, FAM, TET, Yakima Yellow, Eosin, Erythrosin, Dicholorofluorescein, Tetrabromosulfonefluorescein, JOE, Alexa Fluor 488 and 514.

7. A method for detecting a nucleic acid target in a sample by surface-enhanced resonance Raman scattering (SERRS), the method comprising the steps of:
(a) Contacting the sample with an oligonucleotide with a label suitable for SERRS detection, wherein:
- the oligonucleotide essentially consists of bases with neutral nucleosides, and
- the label has a negative charge,
(b) Contacting said probe with a metal surface, and
(c) Detecting the SERRS signal generated by said label on said oligonucleotide.

8. The method according to claim 7, wherein the label is a fluorescent label.

9. The method according to claim 7, wherein the oligonucleotide is a target-specific oligonucleotide.

10. The method according to claim 7, which is a method for detecting multiple targets in a sample, wherein at least two different oligonucleotides each labelled with a different label are contacted with said sample and said detection step comprises detecting the SERRS signal generated by each of said different labels, wherein at least one of said oligonucleotides is **characterised in that:**
- the oligonucleotide consists of bases with neutral nucleosides, and
- the label has a negative charge.

11. The method of claim 10, which comprises detecting the SERRS signal of said different labels at one wavelength.

12. The method of claim 10, which comprises using at least 5 different fluorescent labels.
